(19) Europäisches Patentamt
European Patent Office
Office européen des brevets

(11) Veröffentlichungsnummer: **0 189 096**
**B1**

(12) # EUROPÄISCHE PATENTSCHRIFT

(45) Veröffentlichungstag der Patentschrift: **05.04.89**

(51) Int. Cl.⁴: **C 07 D333/32**

(21) Anmeldenummer: **86100424.0**

(22) Anmeldetag: **14.01.86**

(54) **Verfahren zur Herstellung von Thiotetronsäure.**

(30) Priorität: **16.01.85 CH 192/85**

(43) Veröffentlichungstag der Anmeldung: **30.07.86 Patentblatt 86/31**

(45) Bekanntmachung des Hinweises auf die Patenterteilung: **05.04.89 Patentblatt 89/14**

(84) Benannte Vertragsstaaten: **AT BE CH DE FR GB IT LI LU NL SE**

(56) Entgegenhaltungen:
**TETRAHEDRON, Band 27, Nr. 16, 1971, Seiten 3839-3851, Pergamon Press, Oxford, GB; J.Z. MORTENSEN et al.: "Thiophene chemistry-XVIII. Preparation and tautomeric structures of some potential dihydroxythiophenes"**
**Bericht der deutschen chemischen Gesellschaft 46 (1913) 2103-2107**
**Rodd's Chemistry of carbon compounds, 2nd Ed. vol. IV, Teil A, 245 (1973)**

(73) Patentinhaber: **LONZA AG**
**Gampel/Wallis (CH)**

(72) Erfinder: **Meul, Thomas, Dr. Chem.**
**Balfrinstrasse 21**
**Visp (Kanton Wallis) (CH)**
Erfinder: **Tenud, Leander, Dr. Ing.-Chem.**
**Balfrinstrasse 23**
**Visp (Kanton Wallis) (CH)**

(74) Vertreter: **Weinhold, Peter, Dr. et al**
**Patentanwälte Dr. V. Schmied-Kowarzik Dipl.-Ing. G. Dannenberg Dr. P. Weinhold Dr. D. Gudel Dipl.-Ing. S. Schubert Dr. P. Barz Siegfriedstrasse 8**
**D-8000 München 40 (DE)**

**EP 0 189 096 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft ein neues Verfahren zur Herstellung von Thiotetronsäure.

Thiotetronsäure könnte als Zwischenprodukt zur Herstellung von (±) Thiolactomycin einem Antibiotikum mit breitem Wirkungsspektrum eine bedeutende Anwendungsmöglichkeit finden. Tetrahedron Letters Vol 25 No 46 pp 5243-5246, 1984.

Aus E. Benary, Chem. Berichte 46, 2 103 (1913) ist bekannt die Thiotetronsäure ausgehend von Acetylthioglycoylchlorid durch Reaktion mit Natriummalonester und anschliessendem Ringschluss und Wasserbehandlung herzustellen.

D. B. Macierewicz Rocz. chem. 47, 1735 (1973) hat die Reaktion von E. Benary nachvollzogen und dabei Thiotetronsäure in einer Ausbeute von 30.3 %, bezogen auf das eingesetzte Acetylthioglycoylchlorid erhalten.

Eine andere Möglichkeit zeigt die Synthese von J. Z. Mortensen et al Tetrahedron 27, 3839 (1971). Ausgehend von 2,4 Dibromthiophen erhält er über 3 Stufen durch Umsetzung mit Butyllithium und t-Butylperbenzoat in einer Ausbeute von 46.2 % die Thiotetronsäure.

Bei allen diesen herkömmlichen Synthesen sind die Ausbeute für ein technisches Verfahren viel zu bescheiden.

Ausserdem müssen bei diesen Verfahren deren Umständlichkeit, schwer zugängliche Edukte und schwierig handhabbare Reagenzien in Kauf genommen werden.

Die Aufgabe der vorliegenden Erfindung war es daher, ein Verfahren zur Herstellung der Thiotetronsäure zu finden, das sich durch hohe Ausbeuten und hohe Reinheit der Thiotetronsäure, günstige Edukte und einfache Verfahrensweise auszeichnet.

Erfindungsgemäss wird das dadurch erreicht, dass man 4-Chlor-4-chlormethyloxetan-2-on mit Schwefelwasserstoff in Gegenwart eines Amins direkt zur Thiotetronsäure umsetzt oder die Reaktionslösung vor Isolierung der Thiotetronsäure mit Keten umsetzt, und das Umsetzungsprodukt schliesslich mit einer Mineralsäure zur Thiotetronsäure umsetzt.

Das 4-Chlor-4-chlormethyloxetan-2-on kann auf einfache Wiese gemäss EP Anmeldung 60 808 hergestellt und nach einer Flashdestillation zur erfindungsgemässen Umsetzung eingesetzt werden.

Der Schwefelwasserstoff wird zweckmässig gasförmig eingesetzt.

Als geeignete Amine finden vorteilhaft primäre, sekundäre oder tertiäre Amine, Ammoniak oder auch Guanidine Anwendung.

Als besonders vorteilhaft werden Alkylamine mit niedermolekularen (d. h. 1 bis 6 Kohlenstoffatomen enthaltenden) Alkylgruppen, insbesondere die tertiären Amine wie z. B. Triethylamin, angesehen.

Es ist zweckmässig die Reaktion in Lösungsmitteln durchzuführen. Eingesetzt werden dann gegenüber dem reaktiven Edukt inerte Lösungsmittel wie halogenierte Kohlenwasserstoffe, Ether oder Carbonsäureester. Beispielsweise können Methylenchlorid, Chloroform, etherische Lösungsmittel wie Tetrahydrofuran oder auch Essigester angewendet werden.

Besonders bevorzugt wird aber Tetrahydrofuran verwendet.

Die Edukte werden zweckmässig in einem Mol Verhältnis 4-Chlor-4-chlormethyloxetan-2-on zu Schwefelwasserstoff zu Amin zwischen 1 : 2 : 2 und 1 : 4 : 3 vorzugsweise zwischen 1 : 2,5 : 2 und 1 : 3,5 : 2.5 eingesetzt.

Es wird bevorzugt bei Temperaturen von 0° bis — 40 °C besonders bevorzugt zwischen — 10° und — 25 °C gearbeitet.

Zweckmässig wird so vorgegangen, dass die Eduktlösung mit dem Schwefelwasserstoff gesättigt wird und anschliessend über einen Zeitraum von 30 Minuten bis 120 Minuten das Amin zugegeben wird.

Nach erfolgter Aminzugabe kann die Aufarbeitung der Thiotetronsäure, durch Abfiltrieren vom ausgefallenen Salz und durch darauf folgendes Eindampfen der Lösung erfolgen. Der Rückstand kann zur Abtrennung geringer Mengen dimerer Anhydrothiotetronsäure in etherischen Lösungsmitteln wie Diethylether, THF oder Dioxan vorzugsweise in Diethylether aufgenommen werden und über ein Absorptionsmitteln wie Kieselgel filtriert werden. Nach erneutem Einengen kann die Thiotetronsäure kristallin in guter Ausbeute erhalten werden.

Durch Umkristallisation in aromatischen Kohlenwasserstoffen vorzugsweise in Toluol kann die Thiotetronsäure einer zusätzlichen Reinigung unterzogen werden.

Zur Erzielung einer Thiotetronsäure mit guter Qualität kann nach der Aminzugabe auch so vorgegangen werden, dass ohne Isolation der Roh-Thiotetronsäure diese in Lösung bei Temperaturen von zweckmässig — 10° bis + 5 °C vorteilhaft bei 0 °C mit Keten umgesetzt wird.

Bezogen auf 1 Mol eingesetztes 4-Chlor-4-chlormethyloxetan-2-on wird Keten in Mengen von zweckmässig 2 bis 4 Mol vorzugsweise 2.5 bis 3.5 Mol eingesetzt.

Das Zwischenprodukt der erfindungsgemäßen Umsetzung, das 2,4-Diacetoxythiophen der Formel

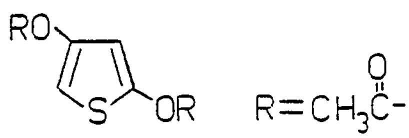

kann gegebenenfalls auf einfache Weise destillativ gereinigt werden.

Durch Behandeln des 2,4-Diacetoxythiophens mit einer nicht oxydierenden anorganischen Mineralsäure erhält man eine reine Thiotetronsäure.

Zweckmässig gelangen Salzsäure, Schwefelsäure vorzugsweise Salzsäure in wässriger Lösung in einer Konzentration von 10 % bis 30 % zur Anwendung.

Die Umsetzungstemperatur liegt zweckmässig zwischen 0 °C und 30 °C vorzugsweise bei 15° bis 25 °C.

Als Reaktionszeit können 2 bis 5 h veranschlagt werden.

Nach Eindampfen vorzugsweise am Hochvakuum erhält man nach dieser Verfahrensweise eine praktisch farblose kristalline Thiotetronsäure in hoher Ausbeute mit einem Gehalt grösser 96 %.

## Beispiel 1

15,5 g (0,091 mol) 4-Chlor-4-chlormethyloxetan-2-on in 300 ml Tetrahydrofuran wurden auf — 20 °C abgekühlt und mit gasförmigem Schwefelwasserstoff gesättigt. Anschliessend gab man tropfenweise, bei — 15 °C, innerhalb einer Stunde eine Lösung von 20,2 g (0,2 mol) Triethylamin, in 100 ml Tetrahydrofuran hinzu. Man liess die Reaktionslösung auf Raumtemperatur erwärmen, filtrierte vom ausgefallenen Salz ab und dampfte das Lösungsmittel am Rotationsverdampfer ab. Der Rückstand wurde über eine mit Kieselgel gefüllte Säule filtriert. Als Laufmittel wurden 300 ml Ether benötigt. Man erhielt 9,0 g gelb gefärbtes, kristallines Produkt mit einem Schmelzpunkt von 115 bis 117 °C. Der Gehalt (HPLC) betrug 89,3 %. Dies entspricht 8,0 g 100 %igem Produkt = 75,7 % Ausbeute. 7,5 g der rohen Thiotetronsäure wurden aus 350 ml Toluol heiss umkristallisiert. Man erhielt 6,6 g schwach, rosa gefärbtes, mikrokristallines Produkt mit einem Schmelzpunkt von 120 °C und einem Gehalt (HPLC) von 96 %. Dies entsprach 6,3 g 100 %igem Produkt ≙ 94 % Ausbeute bzw. 71,2 % auf eingesetztes Oxetanon.

## Beispiel 2

Wie in Beispiel 1 stellte man die Thiotetronsäure her. Man isolierte jedoch die Thiotetronsäure nicht, sondern engte die THF-Lösung bis auf 50 ml ein und leitete bei 0 °C innerhalb einer Stunde 0,3 Mol Keten in diese Lösung ein. Anschliessend liess man die Reaktionslösung auf Raumtemperatur erwärmen und destillierte das Lösungsmittel am Rotationsverdampfer ab. Der Rückstand wurde einer Hochvakuumdestillation unterworfen. Man erhielt 13,7 g 2,4-Diacetoxythiophen GC : 95,6 % $Sdp_{0.25}$ 105 °C, was 13.1 g 100 %igem Produkt entsprach (Ausbeute 65.2 %).

Spektroskopische Daten

$^1$H-NMR (300 MHz, $CDCL_3$) : δ = 2,24 (s,3H), 2,30 (s,3H), 6,57 (d,J) = 2,5Hz,1H, 6,61 (d,1H)

MS (70eV) m/z = 200 ($M^+$,3), 158 ($M^+$—$CH_2$ = C = 0,10), 116 ($M^+$—2 $CH_2$ = C = 0,35), 43 (100)

1,81 g wurden mit 3,5 g 20 %iger Salzsäure versetzt und 3 Stunden bei Raumtemperatur gerührt. Nach 1 Stunde entstand aus der anfänglichen Emulsion eine klare Lösung. Diese gefärbte Lösung wurde im Hochvakuum eingedampft. Man erhielt : 1,0 g praktisch farbloses kristallines Produkt vom Smp. 119 bis 121 °C. (HPLC-Gehalt : 96,1 %). Dies entspricht 0,96 g 100 %iger Thiotetronsäure = 96 % Ausbeute.

**Patentansprüche**

1. Verfahren zur Herstellung von Thiotetronsäure, dadurch gekennzeichnet, dass 4-Chlor-4-Chlormethyloxetan-2-on mit Schwefelwasserstoff in Gegenwart eines Amins direkt zur Thiotetronsäure umgesetzt wird oder die Reaktionsmischung vor Isolierung der Thiotetronsäure mit Keten umgesetzt wird und das Umsetzungsprodukt zum Erhalten der Thiotetronsäure mit einer Mineralsäure umgesetzt wird.

2. Verfahren nach Patentanspruch 1, dadurch gekennzeichnet, dass als Amin primäre, sekundäre oder tertiäre Amine, Ammoniak oder Guanidine angewendet werden.

3. Verfahren nach Patentansprüchen 1 und 2, dadurch gekennzeichnet, dass tertiäre Amine angewendet werden.

4. Verfahren nach Patentansprüchen 1 bis 3, dadurch gekennzeichnet, dass die Reaktion in inerten Lösungsmitteln durchgeführt werden.

5. Verfahren nach Patentansprüchen 1 bis 4, dadurch gekennzeichnet, dass das Eduktverhältnis 4-Chlor-4-chlormethyloxetan-2-on zu Schwefelwasserstoff zu Amin 1 : 2 : 2 bis 1 : 4 : 3 beträgt.

6. Verfahren nach Patentansprüchen 1 bis 5, dadurch gekennzeichnet, dass Temperaturen zwischen 0 °C und — 40 °C angewendet werden.

7. Verfahren nach Patentansprüchen 1 bis 6, dadurch gekennzeichnet, dass Keten in Mengen bezogen auf 1 Mol eingesetzts 4-Chlor-4-chlormethyloxetan-2-on von 2 bis 4 Mol eingesetzt wird.

8. Verfahren nach Patentansprüchen 1 bis 7, dadurch gekennzeichnet, dass als Mineralsäuren nicht oxydierende Mineralsäuren verwendet werden.

## Claims

1. Process for preparing thiotetronic acid, characterized in that 4-chloro-4-chloromethyloxetane-2-one is directly reacted with hydrogen sulfide in the presence of an amine to thiotetronic acid or prior to the isolation of the thiotetronic acid the reaction mixture is reacted with ketene and the reaction product is reacted with a mineral acid to obtain the thiotetronic acid.

2. Process according to patent claim 1, characterized in that as amine primary, secondary or tertiary amines, ammonia or guanidines are used.

3. Process according to patent claims 1 and 2, characterized in that tertiary amines are used.

4. Process according to patent claims 1 to 3, characterized in that the reaction is carried out in inert solvents.

5. Process according to patent claims 1 to 4, characterized in that the educt ratio of 4-chloro-4-chloromethyloxetane-2-one to hydrogen sulfide to amine is 1 : 2 : 2 to 1 : 4 : 3.

6. Process according to patent claims 1 to 5, characterized in that temperatures between 0 and —40 °C are used.

7. Process according to patent claims 1 to 6, characterized in that ketene is used in amounts of 2 to 4 moles, based on 1 mole of 4-chloro-4-chloromethyloxetane-2-one.

8. Process according to patent claims 1 to 7, characterized in that as mineral acids non-oxidizing mineral acids are used.

## Revendications

1. Procédé pour la préparation de l'acide thiotétronique, caractérisé en ce qu'on fait réagir la 4-chloro-4-chlorométhyloxétane-2-one avec de l'hydrogène sulfuré en présence d'une amine directement pour donner l'acide thiotétronique ou en ce qu'on fait réagir le mélange réactionnel avec du cétène avant l'isolement de l'acide thiotétronique et en ce qu'on fait réagir le produit de réaction avec un acide minéral pour obtenir l'acide thiotétronique.

2. Procédé selon la revendication 1, caractérisé en ce qu'on utilise comme amine des amines primaires, secondaires ou tertiaires, l'ammoniac ou la guanidine.

3. Procédé selon les revendications 1 et 2, caractérisé en ce qu'on utilise des amines tertiaires.

4. Procédé selon les revendications 1 à 3, caractérisé en ce que les réactions sont effectuées dans des solvants inertes.

5. Procédé selon les revendications 1 à 4, caractérisé en ce que la proportion de produits de départ 4-chloro-4-chlorométhyloxétane-2-one à l'hydrogène sulfuré à l'amine est de 1 : 2 : 2 à 1 : 4 : 3.

6. Procédé selon les revendications 1 à 5, caractérisé en ce qu'on utilise des températures entre 0 °C et —40 °C.

7. Procédé selon les revendications 1 à 6, caractérisé en ce qu'on met en œuvre le cétène en des quantités de 2 à 4 moles pour une mole de 4-chloro-4-chlorométhyloxétane-2-one mise en œuvre.

8. Procédé selon les revendications 1 à 7, caractérisé en ce que l'on utilise comme acides minéraux des acides minéraux non oxydants.